# EUROPEAN PATENT SPECIFICATION

(11) **EP 2 643 481 B1**
(45) Date of publication and mention of the grant of the patent: **27.04.2016**
(21) Application number: 11842792.1
(22) Date of filing: 17.11.2011
(51) Int. Cl.: G01N 1/30, G01N 33/574, G01N 33/50

(54) **METHODS AND KITS FOR DIFFERENTIAL STAINING OF ABNORMAL URINARY SYSTEM CELLS**
VERFAHREN UND KITS ZUR DIFFERENZIERUNGSFÄRBUNG ABNORMALER HARNSYSTEMZELLEN
PROCÉDÉS ET KITS POUR LA COLORATION DIFFÉRENTIÉE DE CELLULES ANORMALES DU SYSTÈME URINAIRE

(30) Priority: 22.11.2010 US 415893 P
(43) Date of publication of application: 02.10.2013
(73) Proprietor: Zetiq Technologies Ltd., Tel Aviv 61581 (IL)
(72) Inventor: IDELEVICH Pavel, Canton, MA 02021 (US); ELKELES, Adi, 40600 Tel Mond (IL); TERKIELTAUB, Dov, 61581 Tel Aviv (IL); EYAL, Ami, 61581 Tel Aviv (IL)
(74) Representative: Becker Kurig Straus
(86) International application number: PCT/IL2011/050016
(87) International publication number: WO 2012/070041

(56) References cited:
- WO-A2-2007/102146
- WO-A2-2010/081108
- US-A1- 2009 117 610
- IDELEVICH PAVEL ET AL: "Novel Histochemical Stain for Tinctorial Detection of Cancer and Neoplastic Cells", JOURNAL OF HISTOTECHNOLOGY, DOWNERS GROVE, IL, US, vol. 32, no. 3, 1 September 2009 (2009-09-01), pages 97-105, XP009137042, ISSN: 0147-8885
- SONG HE ET AL: "Application of the CellDetect staining technique in diagnosis of human cervical cancer", GYNECOLOGIC ONCOLOGY, vol. 132, no. 2, 1 February 2014 (2014-02-01), pages 383-388, XP055109139, ISSN: 0090-8258, DOI: 10.1016/j.ygyno.2013.12.016
- IAN PROCTOR ET AL: "Biomarkers in bladder cancer", HISTOPATHOLOGY, vol. 57, no. 1, 23 June 2010 (2010-06-23), pages 1-13, XP055022209, ISSN: 0309-0167, DOI: 10.1111/j.1365-2559.2010.03592.x
- KLEIN R.D. ET AL.: 'Transitional Cell Hyperplasia and Carcinomas in Urinary Bladders of Transgenic Mice with Keratin 5 Promoter-Driven Cyclooxygenase-2 Overexpression' CANCER RES. vol. 65, 2005, pages 1808 - 1813, XP055088539

## Description

### FIELD OF THE INVENTION

The present invention relates to methods for identifying abnormal/atypical urinary system cells including, for example, bladder cancer cells.

### BACKGROUND OF THE INVENTION

The urinary system (also called the excretory system or urinary tract) is the system that produces, stores, and eliminates urine. In humans it includes two kidneys, two ureters, the bladder, and the urethra. The urinary bladder is a hollow muscular and distensible organ that collects urine excreted by the kidneys before disposal by urination. Urinary bladder tissue and in particular the epithelial lining of the lower urinary tract (ureters, urinary bladder, and urethra), which is collectively referred to as the urothelium, is susceptible to numerous types of pathologies characterized by abnormal/atypical cells including lesions, tumors and cancers.

Since abnormal urothelial cells can be an indication of urothelial cancer, early identification of such cells and monitoring of diagnosed individuals can be used to prevent or effectively treat life threatening pathologies.

Bladder cancer refers to any of several types of malignant growths of the urinary bladder. It is a disease in which abnormal cells multiply without control in the bladder. The most common type of bladder cancer, transitional cell carcinoma (TCC), begins in the urothelium.

Carcinoma of the urinary bladder occurs with an incidence of 15 cases per 100,000 persons in the general population. It is the fourth most common cancer in males and its frequency is 3 times higher in males than in females. Over 90% of newly diagnosed bladder cancers are transitional cell carcinomas. The 5-year survival rate is ~95% when the tumor is confined to the bladder, and decreases to 46% and 6% when spreading locally or to distant sites, respectively, emphasizing the need for an effective early detection tool.

The current "gold standard" for detection of bladder cancer includes urine cytology and cystoscopy. Cystoscopic examination is an invasive test and is associated with relatively high costs, and urine cytology has low sensitivity, particularly in low-grade superficial TCC. Many clinical studies are underway with the aim of introducing new molecular markers in urine samples for the detection of TCC. These tests are potentially costly and thus pose a challenge for applicability as a general screening tool.

Thus, there is a need for an accurate method of detecting bladder cancer, especially in the early stages thereof.

Idelevich Pavel et al. ("Novel histochemical stain for tinctorial detection of cancer and neoplastic cells"; Journal of Histotechnology, September 1, 2009, Downers Grove, Il, USA; Vol. 32, No. 3, p. 97-105) discloses three kits developed by Zetiq Technologies Ltd. collectively termed CellDetect®. Further disclosed is the use of these kits for staining cells and clinical material, including a urinary bladder tissue. The CellDetect® kits contain three ingredients: 1) an acidophilic red stain, 2) a basophilic green stain, and 3) a plant extract.

### SUMMARY OF THE INVENTION

According to one aspect of the present invention there is provided a method of identifying abnormal cells in a urine cell sample, the method comprising (a) fixing the urine sample comprising urinary system cells in trichloroacetic acid; (b) contacting the urine sample with a Ficus plant extract or one or more components thereof, thereby obtaining conditioned abnormal urinary system cells; (c) staining said conditioned urinary system cells with a basic dye and an acidic dye, such that a cytoplasm of the abnormal cells of the urinary system cells stains with a predominantly pink or red color.

According to embodiments of the present invention, the Ficus plant is from the subgenus Urostigma, and is preferably Ficus elastica.

According to embodiments of the present invention, the Ficus plant extract is an ethanol extract of leaf tissue.

According to embodiments of the present invention, the basic dye is New Fuchsin and the acidic dye is Light Green or Fast green.

According to embodiments of the present invention, staining conditioned urinary system cells with New Fuchsin is followed staining the cells with Light Green or Fast green.

According to embodiments of the present invention, the urinary system cells are urothelium cells; or the urinary system cells are bladder cells.

According to embodiments of the present invention, the one or more components of the extract include one or more flavonoids; or the one or more components of the extract include one or more flavonoids and the one or more flavonoids include proanthocyanidins.

According to embodiments of the present invention, the urinary system cells are urothelial cells, the conditioned cells are sequentially stained with the basic dye and the acidic dye, and identifying at least one urothelial cell having a cytoplasm having a basic dye color above a pre-determined threshold is indicative of abnormal cells in the sample.

According to embodiments of the present invention, the urinary system cells are bladder cells, and identifying at least one bladder cell having a red cytoplasm above a pre-determined threshold is identifying cell abnormalities indicative of the bladder pathology.

According to embodiments of the present invention, the method further comprises analyzing a morphology of the at least one bladder cell having a predominantly pink to red cytoplasm, wherein the conditioned cells are sequentially stained with the basic dye and the acidic dye, wherein the Ficus is Ficus elastica

According to embodiments of the present invention, the method further comprises contacting the bladder cells with an immunostain; or contacting the bladder cells with an anti-Ki67 immunostain

According to embodiments of the present invention, the bladder pathology is bladder cancer; or the bladder pathology is transitional cell carcinoma.

The present invention successfully addresses the shortcomings of the presently known configurations by providing an accurate and simple approach for detecting abnormal cells in a urinary system of a subject.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.
FIGs. 1a-d illustrate normal bladder epithelium stained with hematoxylin and eosin (H&E), magnified 40X (Figures 1a and 1c), or with the present staining approach (Figures 1b and 1d). As shown in these figures, normal bladder epithelium stained with the present approach exhibits a uniform green cytoplasmic color.
FIGs. 2a-d illustrate low grade transitional cell carcinoma (TCC) tissue stained with H&E (Figures 2a and 2c) or the present approach (Figures 2b and 2d); magnified 40X. The cytoplasm of the TCC cells exhibits a unique reddish/violet color when stained with the present approach.
FIGs. 3a-d illustrate high grade TCC tissue stained with H&E (Figures 3a and 3c) or the present approach (Figures 3b and 3d);magnified 40X. The cytoplasm of the TCC cells exhibits a unique reddish color when stained with the present approach.

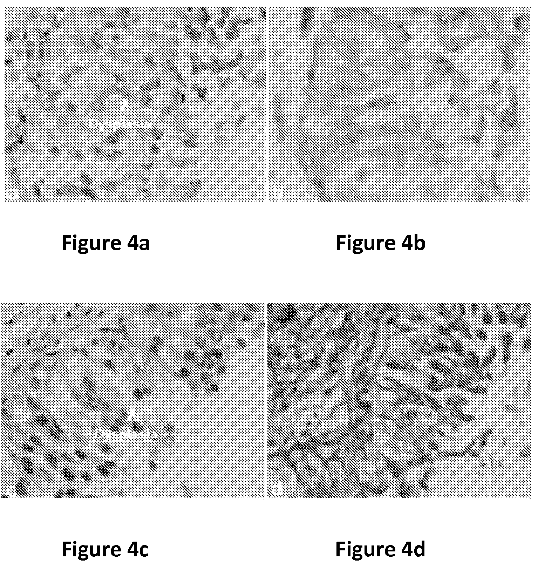

FIGs. 4a-d illustrate dysplasia of bladder epithelium stained with H&E (Figures 4a and 4c) or the present approach (Figures 3b and 3d); magnified 40X.The cytoplasm of dysplastic cells exhibits a pink color when stained with the present approach.
FIGs. 5a-d illustrate low grade TCC tissue stained with the present approach (Figures 5a and 5c) or Ki67 immunostain (Figures 5b and 5d), magnified 40X. Cell areas stained in red (Figure 5a) have a high Ki67 index (Figure 5b), while cell areas stained in green (Figure 5c) have relatively low Ki67 index (Figure 5d).
FIGs. 6a-b illustrate low grade TCC tissue stained with the present approach (Figure 6a) or Ki67 (Figure 6b), magnified 10X.The cell cytoplasm is stained with a mix of red and green (Figure 6a) and shows relatively low Ki67 index (Figure 6b).
FIGs. 7a-d illustrate normal bladder epithelium (Figures 7a-b) and low grade TCC (Figures 7c-d) stained with H&E (Figures 7a and 7c) or double stained with Ki67 and the present approach (Figures 7b and 7d); magnified 40X. Normal tissue exhibits a blue/green cytoplasmic stain (Figure 7a) and is devoid of Ki67-positive cells (Figure 7b). The low grade TCC tissue exhibits a uniform pink cytoplasmic stain (Figure 7c) and includes numerous Ki67 positive cells (Figure 7d).
FIGs. 8a-b illustrate urinary cytology of normal and high grade TCC cells isolated from urine samples, and stained with the present approach; magnified 40X. Cytoplasms of normal epithelial cells (and inflammatory cells) are stained blue/green (Figure 8a), while cytoplasms of high grade TCC cells are stained red/pink (Figure 8b).

### DESCRIPTION OF SPECIFIC EMBODIMENTS OF THE INVENTION

The present invention relates to methods for detecting abnormal urinary system cells and use thereof in diagnosing a pathology such as bladder cancer.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

Bladder cancer (e.g. TCC, squamous cell carcinoma, and adenocarcinoma) is the fifth most common cancer in U.S. TCC is by far the most common form of bladder cancer accounting for more than 90% of bladder cancer cases in the U.S. Bladder cancer is currently diagnosed using cystoscopy and cytology in patients with suspicious symptoms.

A number of urine based tumor markers for identifying malignant cells in the urine are known. However, diagnosis of bladder cancer using such markers requires additional visualization of a tumor via cystoscopy and confirmation by transurethral resection or biopsy. In addition, such markers are highly insensitive to low-grade bladder neoplasms.

PCT Publication No. WO2007/102146 to the present inventors discloses a method of staining or pre-staining cells using an extract of a Ficus elastica plant, or active ingredients thereof, for detection of cell abnormalities resulting from cancer or metabolic diseases.

While reducing the present invention to practice, the staining approach described in WO2007/102146 was modified and adapted for use in distinguishing between normal and abnormal urothelial cells (e.g. TCC cells). As is clearly shown in Example 1 of the Examples section which follows, the staining approach of the present invention (also referred to herein as "the present approach") can accurately and rapidly identify even low grade TCC cells and is thus highly suitable for use in early detection of urinary system pathologies such as bladder cancer.

Moreover, the staining methods of the invention provide a highly advantageous diagnostic platform of high accuracy and specificity for identifying bladder cancer in urine samples. This approach addresses the need for a non invasive diagnosis of bladder abnormalities.

Thus, according to one aspect of the present invention there is provided a method of identifying abnormal cells in a urine cell sample.

As used herein the phrase "urinary system cells" refers to cells (e.g. epithelial cells) of a kidney, ureter, urethra or bladder.

The cells can be provided for analysis as a cell suspension or a smear.

A smear can be a crude sample of urinary system cells smeared on a microscopic slide without any purification. Other methods for preparation of urine cells for cytology, include, but are not limited to, cytospin or liquid based cytology methods such as ThinPrep™ (Hologic, USA).

Urinary system cells can also be cultured prior to conditioning and staining. Such culturing can be effected using methods known in the art, see for example, Hertz et al. "Short-term culture of exfoliated cells from the urine of patients with bladder tumors", Urological Research, Volume 21, Number 1, 23-26, 1993; and Belik et al. "Improvements in Culturing Exfoliated Urothelial Cells In Vitro from Human Urine" Journal of Toxicology and Environmental Health, Part A, Volume 71, Issue 13 and 14 January 2008, pages 923 - 929.

In order to increase detection accuracy, the cell sample can be pretreated to remove non-urinary system cells, such as circulatory system cells (e.g. leukocytes and erythrocytes) and the like, using method well known in the art of cell separation (e.g. size exclusion, selective cell lysis etc.).

The method is effected by contacting the urinary system cells with a Ficus plant extract or one or more components thereof thereby conditioning the abnormal cells for identification.

As used herein, the term "conditioning" when used in reference to cells or tissues refers to the step of rendering cells (and in particular abnormal cells) more amenable to subsequent detection via histological staining or via biochemical or molecular approaches. The conditioning step of the present invention is preferably effected using an ethanol extract of Ficus Elastica leaf tissue.

Contacting the urinary system cell sample with the Ficus plant extract or components thereof can be performed by applying it on the urinary system cell sample, or by dipping, soaking and/or incubating the urinary system cell sample in a vessel containing same.

Such contacting is effected for a time period which enables conditioning of abnormal cells such that they will be detectable via one of the detection approaches described herein. Contacting can be performed for about 1-20 minute or more under ambient conditions or elevated heat and pressure.

As used herein, the phrase "Ficus plant extract" refers to an ethanol/methanol extract from whole or cut/ground plant tissue (e.g. leaves) of a Ficus plant preferably from the subgenus Urostigma, more preferably of the Ficus elastica species. The ethanol extract of the plant tissue can include about 10 % (v/v ethanol in water), about 20 %, about 30 %, about 40 %, about 50 % ethanol, about 60 % ethanol, about 70 % ethanol, about 80 % ethanol about 90 % ethanol or about 100 % ethanol. Measures should be taken however not to over dilute the extract, as this may affect the subsequent staining. Exemplary extraction protocols are described below.

The extract is produced as follows. Dark green leaves, between 15 and 25 cm in length (as measured from the base of the leaf to the tip) are obtained from Ficus elastica. A 1000 gr sample of leaves is cut into 1-3 cm² pieces and washed with deionized water (DIW). Leaf pieces are mixed with 3 liters of 70% ethanol and kept for 14 days (room temperature) in a sealed container, in the dark. Thereafter, the liquid is separated from the solids and maintained for further use at room temperature.

Alternatively, the leaf pieces are dried in an oven to a water content of about 4% (65°C for 24 hours) and the dry leaf material is blended into a powder (700-1000 micron). The powder is then used in extraction in a reflux system: 1 hour per extraction, solvent: 70% ethanol, 40°C. Three sequential extractions are performed, each time the powder is reextracted. Quantity of 70% ethanol in each extraction: first - 1:5; second - 1:4; third - 1:3. The three resulting extracts are mixed, filtered in vacuum filter (paper filter No. 40). The mixed, filtered extract is evaporated in a rotor evaporator under vacuum at 60°C until a steady weight is obtained. The resulting powder (final water content - 3%) is blended further in a grinder. The powder extract is reconstituted for use as a conditioning reagent. The powder is suspended in 70% ethanol with 1.3% TDS w/v and a final pH of 7.4.

Components of the plant extract are preferably the active ingredients of the plant [e.g., flavonoids in an oligomeric form (which may comprise 2-10 mers, e.g., C₂₆H₃₂O₁₅) or a polymeric form, C₂₃H₄₄O₄ or proanthocyanidins], which provide the characteristic conditioning abilities described herein. Such components can be isolated from the plant extract or synthesized.

As is mentioned hereinabove, the conditioning step of the present invention can be followed by a detection step which is effected using biochemical, molecular or cell/tissue staining approaches.

For detection, the cell/tissue sample can be prepared on a slide or used in suspension. Methods of attaching cells to microscopic slides are well known in the art, and include, for example, layering cells (using smear or liquid-based cytology, e.g. ThinPrep^{™}) of the microscopic slides, centrifuging the cells on the slide (e.g., using a cytospin), mounting tissue sections on the slides (e.g., using paraffin-embedded sections), or smearing cells over a microscopic slide.

The microscopic slides which include the cell sample can be used per-se or can be precoated with agents which increase the adhesiveness of the cells to the slide (e.g., poly-L-lysine or silane). The slides can be heated, frozen or subjected to energy of a certain wave-length (e.g., U.V.) in order to increase adhesiveness of the cells to the slide.

It should be noted that the urinary system cell sample is fixed with a fixative prior to detection. Methods of fixing cells are well known in the art, and include the use of trichloroacetic acid.

Cell suspensions can be analyzed in containers such as dishes, flasks, tubes and the like. The cells can be suspended in DIW or TRIS-buffered saline (TBS).

Analysis of slides or suspensions can be effected using subjective approaches (e.g. pathologist described in the Examples section which follows), or by using objective approaches (e.g. automated screening) using, for example, optical (image) analysis software. Since the preferred staining approach of the present invention relies upon color differences (between normal and abnormal cells) as well as differences in morphology, automated analysis can employ computer algorithms designed for automatic detection of such color differences as well as color density/texture, size of color region and the like. One example of an algorithm which can detect cells based on differential staining/color is THREECOND. An optical detection algorithm can be combined with morphometric detection algorithms in order to improve the reliability of the results.

The present approach can also include a step of immunocytological screening. In recent years, several non-invasive tests for tumor markers have been approved by the U.S. Food and Drug Administration (FDA). Commercially available tests for the diagnosis of bladder cancer include ImmunoCyt/uCyt+, BTA TRAK®, BTA stat®, NMP22®, NMP22 Bladder- Chek®, and UroVysion™. Such tests can be used to further validate results obtained by the present approach if necessary.

Staining of the cell sample is performed using staining agents (e.g., dyes) and conditions which enable unique or differential staining of abnormal cells. Preferred are basic and acidic dyes and combinations thereof.

As used herein the phrase "differential staining" refers to staining an abnormal cell or a cell compartment thereof (e.g., cytoplasm) with a different color or tone than a normal cell of the same type.

Examples of dyes that can be used to stain the conditioned cell sample include, but are not limited to, the following dyes (Catalog numbers indicated are from StainsFile (http://stainsfile.info/StainsFile/jindex.html): acetyl yellow 13015, Nitro Fast yellow, Acid black 1 20470, AzoAmido black 10B, Acid blue 22 42755, Triarylmethane, Water blue I, Acid blue 93 42780, Triarylmethane, Methyl blue, Acid fuchsin 42685, Triarylmethane Acid fuchsin Acid green 42095, Triarylmethane, Light Green SF yellowish, Acid green 1 10020, NitrosoNaphthol green B, Acid green 5 42095, Triarylmethane Light Green SF yellowish Acid magenta 42685, Triarylmethane, Acid fuchsin, Acid orange 10 16230, Azo Orange G, Acid red 4 14710, AzoAzo-eosin, Acid red 26 16150, AzoXylidineponceau, Acid red 29 16570, AzoChromotrope 2R, Acid red 44 16250, AzoPonceau 6R, Acid red 51 45430, FluoroneErythrosin B, Acid red 66 26905, AzoBiebrich scarlet, Acid red 73 27290, AzoWoodstain scarlet, Acid red 87 45380, Fluorone Eosin Y ws, Acid red 91 45400, Fluorone Eosin B, Acid red 92 45410, FluoronePhloxine B, Acid red 94 45440, Fluorone, Rose bengal, Acid red 101 50085, Quinone-Imine, Azocarmine G, Acid red 103 50090, Quinone-Imine Azocarmine B, Acid roseine 42685, Triarylmethane Acid fuchsin Acid rubin 42685, Triarylmethane Acid fuchsin Acid violet 19 42685, Triarylmethane Acid fuchsin Acid yellow 1 10316, Nitro Naphthol yellow S, Acid yellow 9 13015, Nitro Fast yellow, Acid yellow 23 19140, AzoTartrazine, Acid yellow 24 10315, Nitro Martius yellow, Acid yellow 36 13065, AzoMetanil yellow, Acid yellow 73 45350, Fluorone Fluorescein Acid yellow S 10316, Nitro Naphthol yellow S, Acid yellow T 19140, AzoTartrazineAcridine orange 46005, AcridineAcridine orange Acriflavine 46000, AcridineAcriflavine Alcian blue 74240, Phthalocyanine Alcian blue 8GX, Alcian yellow 12840, Azo Alcian yellow, Alcohol soluble 45386, Fluorone Ethyl eosin eosin Alizarin 58000, Anthraquinone Alizarin, Alizarin blue 67410, Anthraquinone Alizarin blue Alizarin blue 2RC 58605, Anthraquinone Anthracene blue SWR, Alizarin carmine 58005, Anthraquinone, Alizarin red S, Alizarin cyanin BBS 58610, Anthraquinone Alizarin cyanin BBS, Alizarolcyanin R 43820, TriarylmethaneChromoxanecyanin R Alizarin red S 58005, Anthraquinone Alizarin red S Alizarin purpurin 58205, AnthraquinonePurpurinAluminon 43810, Triarylmethane Chrome violet CG Amido black 10B 20470, AzoAmido black 10B Amidonaphthol red 18050, AzoAzophloxineAmidoschwarz 20470, AzoAmido black 10B Aniline blue WS - Triarylmethane Aniline blue WS Aniline purple -- AzinMauveine Anthracene blue SWR 58605, Anthraquinone Anthracene blue SWR Anthracene blue SWX 58610, Anthraquinone Alizarin cyanin BBS Auramine O 41000, DiarylmethaneAuramine O Azo-eosin 14710, AzoAzo-eosin Azocarmine B 50090, Quinone-Imine Azocarmine B Azocarmine G 50085, Quinone-Imine Azocarmine B Azoeosin G 14710, AzoAzo-eosin Azoic diazo 5 37125, Diazonium salt Fast red B Azoic diazo 48 37235, Diazonium salt Fast blue B Azophloxine 18050, AzoAzophloxineAzovan blue 23860, Azo Evans blue Azure A 52005, Thiazin Azure A Azure B 52010, Thiazin Azure B Azure C 52002, Thiazin Azure C Basic blue 8 42563, Triarylmethane Victoria blue 4R Basic blue 9 52015, Thiazin Methylene blue Basic blue 12 51180, Oxazin Nile blue A Basic blue 15 44085, Triarylmethane Night blue Basic blue 17 52040, Thiazin Toluidine blue O Basic blue 20 42585, Triarylmethane Methyl green Basic blue 26 44045, Triarylmethane Victoria blue B Basic brown 1 21000, Azo Bismarck brown Y Basic fuchsin -- Triarylmethane Basic fuchsin Basic green 4 42000, Triarylmethane Malachite green Basic green 5 52020 Thiazine Methylene green Basic orange 14 46005, AcridineAcridine orange Basic red 2 50240, SafraninSafranin O Basic red 5 50040, Eurhodin Neutral red Basic red 9 42500, TriarylmethanePararosanilin Basic violet 2 42520, Triarylmethane New fuchsin Basic violet 3 42555, Triarylmethane Crystal violet Basic violet 4 42600, Triarylmethane Ethyl violet Basic violet 10 45170, RhodamineRhodamine B Basic violet 14 42510, TriarylmethaneRosanilin Basic yellow 1 49005, ThiazoleThioflavine T Basic yellow 2 41000, DiarylmethaneAuramine O Biebrich scarlet 26905, AzoBiebrich scarlet Biebrich scarlet R 26105, Azo Sudan IV Bismarck brown Y 21000, Azo Bismarck brown Y Brazilein 75280, Natural Brazilein Brazilin 75280, Natural Brazilin Brilliant crocein 27290, AzoWoodstain scarlet Brilliant crystal 16250, AzoPonceau 6R scarlet 6R Calcium red 60760, Anthraquinone Nuclear fast red Carmine 75470, Natural Carmine Carminic acid 75470, Natural Carmine Carmoisine 6R 16570, AzoChromotrope 2R Celestine blue B 51050, Oxazin Celestine blue B China blue -- -- Aniline blue Chlorantine fast 28160, Azo Sirius red 4B red 5B Cochineal 75470, Natural Carmine Coelestine blue 51050, Oxazin Celestine blue B Chicago blue 4B -- azoPontamine sky blue 5B Chrome violet CG 43810 ,Triarylmethane Chrome violet CG Chromotrope 2R 16570 AzoChromotrope 2R Chromoxanecyanin R 43820 TriarylmethaneChromoxanecyanin R Congo corinth 22145, Azo Congo corinth Congo red 22120, Azo Congo red Cotton blue 42780, Triarylmethane Methyl blue Cotton red 22120, Azo Congo red Croceine scarlet 26905, AzoBiebrich scarlet Crocein scarlet 3B 27290, AzoWoodstain scarlet Crocein scarlet MOO 27290, AzoWoodstain scarlet Crocin 75100, Natural Saffron Crystal ponceau 6R 16250, AzoPonceau 6R Crystal scarlet 16250, AzoPonceau 6R Crystal violet 42555, Triarylmethane Crystal violet Dahlia 42530, Triarylmethane Hoffman's violet Diamond green B 42000, Triarylmethane Malachite green Direct blue 14 23850, AzoTrypan blue Direct blue 58 23860, Azo Evans blue Direct red 22120, Azo Congo red Direct red 10 22145 Azo Congo corinth Direct red 28 22120, Azo Congo red Direct red 80 35780, Azo Sirius red F3B Direct red 81 28160, Azo Sirius red 4B Direct yellow 7 49010, ThiazoleThioflavine S Durazol blue 4R -- AzoDurazol blue 4R Durazol blue 8G -- PhthalocyanineDurazol blue 8G Eosin B 45400, Fluorone Eosin B Eosin Bluish 45400, Fluorone Eosin B Eosin 45380, Fluorone Eosin Y ws Eosin Y 45380, Fluorone Eosin Y ws Eosin yellowish 45380, Fluorone Eosin Y wsEosinol -- FluoroneEosinol Erie garnet B 22145, Azo Congo corinthEriochromecyanin R 43820, TriarylmethaneChromoxanecyanin R Erythrosin B 45430, FluoroneErythrosin B Ethyl eosin 45386, Fluorone Ethyl eosin Ethyl green 42590, Triarylmethane Ethyl green Ethyl violet 42600, Triarylmethane Ethyl violet Evans blue 23860, Azo Evans blue Fast blue B 37235, Diazonium salt Fast blue B Fast green FCF 42053, Triarylmethane Fast green FCF Fast red B 37125, Diazonium salt Fast red B Fast yellow 13015 Nitro Fast yellow Fast yellow extra 13015, Nitro Fast yellow Fast yellow G 13015, Nitro Fast yellow Fat black HB 26150, Azo Sudan black B Fluorescein 45350, Fluorone Fluorescein Food green 3 42053, Triarylmethane Fast green FCF Gallein 45445, FluoroneGalleinGallamine blue 51045, OxazinGallamine blue Gallocyanin 51030, OxazinGallocyanin Gentian violet -- Triarylmethane Methyl violet 2B Haematein 75290, Natural HemateinHaematine 75290, Natural Hematein Haematoxylin 75290, Natural Hematoxylin Helio fast rubin BBL 60760, Anthraquinone Nuclear fast red Helvetia blue 42780, Triarylmethane Methyl blue Hematein 75290, Natural HemateinHematine 75290, Natural Hematein Hematoxylin 75290, Natural Hematoxylin Hoffman's violet 42530, Triarylmethane Hoffman's violet Hydrazine yellow 19140, AzoTartrazine Imperial red 45400 Fluorone Eosin B Ingrain blue 1 74240, Phthalocyanine Alcian blue 8GX Ingrain yellow 1 12840, Azo Alcian yellow INT -- TetrazoliumIodonitrotetrazolium salt Kermes 75460 Natural Kermes Kermesic acid 75460, Natural Kermes Kernechtrot 60760, Anthraquinone Nuclear fast red Lac 75450 Natural Laccaic acid Laccaic acid 75450, Natural Laccaic acid Lauth's violet 52000, ThiazinThionin Light Green 42095, Triarylmethane Light Green SF yellowish Lissamine fast 18965, AzoLissamine fast yellow yellowLissamine green SF 42095, Triarylmethane Light Green SF yellowish Luxol fast blue -- PhthalocyanineLuxol fast blue MBS Magenta 0 42500, TriarylmethanePararosanilin Magenta I 42510, Triarylmethane Rosanilin Magenta II -- Triarylmethane Magenta II Magenta III 42520, Triarylmethane New fuchsin Malachite green 42000, Triarylmethane Malachite green Manchester brown 21000, Azo Bismarck brown Y Martius yellow 10315, Nitro Martius yellow Mauve-AzinMauveineMauveine -- AzinMauveineMerbromin -- Fluorone Mercurochrome 220, Mercurochrome -- Fluorone Mercurochrome 220, Metanil yellow 13065, AzoMetanil yellow Methylene azure A 52005 Thiazin Azure A Methylene azure B 52010, Thiazin Azure B Methylene azure C 52002, Thiazin Azure C Methylene blue 52015, Thiazin Methylene blue Methylene green 52020, Thiazine Methylene green Methyl blue 42780, Triarylmethane Methyl blue Methyl green 42585, Triarylmethane Methyl green Methyl violet 42535 Triarylmethane Methyl violet 2B Methyl violet 2B 42535, Triarylmethane Methyl violet 2B Methyl violet 10B 42555, Triarylmethane Crystal violet Milling yellow 3G -- Azo Milling yellow 3G Mordant blue 3 43820 TriarylmethaneChromoxanecyanin R Mordant blue 10 51030, OxazinGallocyanin Mordant blue 14 51050, Oxazin Celestine blue B Mordant blue 23 58610, AnthraquinoneAlizarin cyanin BBS Mordant blue 32 58605, Anthraquinone Anthracene blue SWR Mordant blue 45 51045, OxazinGallamine blue Mordant red 3 58005 Anthraquinone Alizarin red S Mordant red 11 58000, Anthraquinone Alizarin Mordant violet 25 45445, FluoroneGallein Mordant violet 39 43810, Triarylmethane Chrome violet CG Naphthalene blue -- AzoNaphalene blue black blackNaphthol blue black 20470 AzoAmido black 10B Naphthol green B 10020, NitrosoNaphthol green B Naphthol yellow S 10316, Nitro Naphthol yellow S Natural black 1 75290, Natural Hematein Natural red 58205, AnthraquinonePurpurin Natural red 3 75460, Natural Kermes Natural red 4 75470, Natural Carmine Natural red 8 58205, Anthraquinone Purpurin Natural red 16 58205, AnthraquinonePurpurin Natural red 24 75280, Natural Brazilin Natural red 25 75450, Natural Laccaic acid Natural red 28, -- Natural Orcein Natural yellow 6 75100, Natural Saffron NBT -- Tetrazolium Nitro blue salt tetrazolium Neutral red 50040, Eurhodin Neutral red New fuchsin 42520, Triarylmethane New fuchsin Niagara blue 3B 23850, AzoTrypan blue Night blue 44085, Triarylmethane Night blue Nile blue 51180, Oxazin Nile blue A Nile blue A 51180, Oxazin Nile blue A Nile blue sulphate 51180, Oxazin Nile blue A Nile red -- Oxazone Nile red Nitro BT -- Tetrazolium Nitro blue salt tetrazolium Nitro blue -- Tetrazolium Nitro blue tetrazolium salt tetrazolium Nuclear fast red 60760, Anthraquinone Nuclear fast red Oil red O 26125, Azo Oil red O Orange G 16230, Azo Orange G Orcein -- Natural OrceinPararosanilin 42500 TriarylmethanePararosanilin Perkin's violet -- AzinMauveinePhloxine B 45410, FluoronePhloxine B Picric acid 10305 Nitro Picric acid Ponceau 2R 16150, AzoXylidineponceauPonceau 6R 16250, AzoPonceau 6R Ponceau B 26905, AzoBiebrich scarlet Ponceau de Xylidine 16150, AzoXylidineponceauPonceau S 27195, AzoPonceau S Pontamine sky -- azoPontamine sky blue 5B blue 5B Primula 42530, Triarylmethane Hoffman's violet Pumuline 49000, ThiazolePrimulinePurpurin 58205, AnthraquinonePurpurinPyronin B 45010, PyroninPyronin B Pyronin G 45005, PyroninPyronin Y Pyronin Y 45005 PyroninPyronin Y Rhodamine B 45170 RhodamineRhodamine B Rosanilin 42510, TriarylmethaneRosanilin Rose bengal 45440, Fluorone Rose bengal Saffron 75100, Natural Saffron Safranin O 50240, SafraninSafranin O Scarlet R 26105, Azo Sudan IV Scarlet red 26105, Azo Sudan IV Scharlach R 26105, Azo Sudan IV Shellac 75450, Natural Laccaic acid Sirius red F3B 35780, Azo Sirius red F3B Sirius red 4B 28160, Azo Sirius red 4B Sirius supra -- AzoDurazol blue 4R blue F3R SolochromecyaninR 43820, TriarylmethaneChromoxanecyanin R Soluble blue - Aniline blue Solvent black 3 26150, Azo Sudan black B Solvent blue 38 - PhthalocyanineLuxol fast blue MBS Solvent red 23 26100, Azo Sudan III Solvent red 24 26105, Azo Sudan IV Solvent red 27 26125, Azo Oil red O Solvent red 45 45386, Fluorone Ethyl eosin Solvent yellow 94 45350, Fluorone Fluorescein Spirit soluble 45386, Fluorone Ethyl eosin eosin Sudan III 26100, Azo Sudan III Sudan IV 26105, Azo Sudan IV Sudan black B 26150, Azo Sudan black B Sudan red BK 26100 Azo Sudan III Sulfur yellow S 10316, Nitro Naphthol yellow S Swiss blue 52015 Thiazin Methylene blue Tartrazine 19140, AzoTartrazineThioflavine S 49010, ThiazoleThioflavine S Thioflavine T 49005 ThiazoleThioflavine T Thionin 52000, ThiazinThionin Toluidine blue 52040, Thiazin Toluidine blue O Toluyline red 50040, Eurhodin Neutral red Tropaeolin G 13065, AzoMetanil yellow Trypaflavine 46000, AcridineAcriflavineTrypan blue 23850, AzoTrypan blue Uranin 45350, Fluorone Fluorescein Victoria blue 4R 42563, Triarylmethane Victoria blue 4R Victoria blue B 44045, Triarylmethane Victoria blue B Victoria blue R 44040, Triarylmethane Victoria blue R Victoria green B 42000, Triarylmethane Malachite green Water blue I 42755, Triarylmethane Water blue I Water soluble eosin 45380, Fluorone Eosin Y wsWoodstain scarlet 27290, AzoWoodstain scarlet Xylidineponceau 16150, AzoXylidineponceau Yellowish eosin 45380, Fluorone Eosin Y ws.

Preferred dyes include the basic dye New Fuchsin, the cationic dye Alcian Blue, and the acidic dyes Light Green or Fast Green.

New Fuchsin stains abnormal urinary system cells - pre-conditioned as described herein - with an intense red color, while leaving normal cell stained with a lighter less intense tone of red (light red or pink). Such staining concentrates at the nucleus and cytoplasm and is indicative of an abnormal phenotype.

New Fuchsin is available as powder from various manufacturers such as Sigma (Cat. No. N0638), Fluka (Cat. No. 72200) or Merck (Cat. No. 1052260100).

The preferred concentration of New Fuchsin (for fixed and mounted tissue) can be about 0.05-0.5 % (w/v) in a solution of 2-20 % [volume per volume (v/v)] ethanol in water.

New Fuchsin can be used at a concentration of about 0.02 % [weight per volume (w/v)], about 0.1 % (w/v), about 0.2 % (w/v), about 0.3 % (w/v), about 0.4 % (w/v), about 0.5 % (w/v), about 0.6 % (w/v) or about 0.7 % (w/v).

A New Fuchsin solution can be water and/or ethanol-based and can include about 2 % ethanol, about 5 % ethanol, about 10 % ethanol, about 15 % ethanol, about 20% ethanol, about 25 % ethanol, about 30 % ethanol, about 35 % ethanol, about 40 % ethanol or about 50 % ethanol.

Staining the conditioned urinary system cell sample with New Fuchsin can be performed by applying the New Fuchsin on the urinary system cell sample, adding it thereto, or by dipping, soaking and/or incubating the urinary system cell sample in a vessel containing same. Staining the urinary system cell sample with New Fuchsin can be effected for a time period which enables differential staining of abnormal cells by New Fuchsin, e.g., at least 5 seconds, at least about 10 seconds, at least about 1 minute, about 1-10 minutes.

Alcian Blue may be used by the present invention in order to reduce the level of non-specific background staining of normal urinary cells or to stain mucins in case of Mucinuria. Moreover, Alcian Blue can serve as a mordant of Light Green, known in its rapid fading, enabling delay of decoloration.

Alcian Blue is available as powder from various manufacturers such as Sigma (Cat. No. 5500) or Merck (Cat. No. 1052340010).

The preferred concentration of Alcian Blue (for fixed and mounted tissue) can be about 0.05-0.5 % (w/v) in water.

Alcian Blue can be used at a concentration of about 0.05% [weight per volume (w/v)], about 0.1 % (w/v), about 0.2 % (w/v), about 0.3 % (w/v), about 0.4 % (w/v), about 0.5 % (w/v).

Staining the urinary system cell sample with Alcian Blue can be performed by applying the Alcian Blue to the cell sample or by dipping, soaking and/or incubating the cell sample in a vessel containing this dye. Staining the urinary system cell sample with Alcian Blue can be effected for a time period which enables reducing of non-specific background, staining of Mucins and protecting Light Green from fading (e.g., at least 30 seconds).

Preferably, staining with Alcian Blue is effected following staining with New Fuchsin.

The urinary system cell sample can be alternatively or preferably additionally stained with Light Green or Fast green.

Light Green and fast Green are acidic dyes which stain the cytoplasm of normal cells with green (leaving the cytoplasm of abnormal cells red/pink/purple following New Fuchsin staining). Light Green is available in a powder from various manufacturers such as Merck (C.I. 42095) or Sigma (Cat. No. 62110). Fast green is available from Sigma-Aldrich (F7258) in a form of a powder.

Light Green can be used at a concentration of about 0.02 % [weight per volume (w/v)], about 0.1 % (w/v), about 0.2 % (w/v), about 0.3 % (w/v), about 0.4 % (w/v), about 0.5 % (w/v), about 0.6 % (w/v), about 0.7 % (w/v) about 0.8 % (w/v), about 1 % (w/v), about 2 % (w/v), about 3 % (w/v), about 4 % (w/v), about 5 % (w/v), about 6 % (w/v), about 7 % (w/v), about 8 % (w/v), about 9 % (w/v) or about 10 % (w/v).

The Light Green solution can be water and/or ethanol-based, and can include 1% ethanol, about 5 % ethanol, about 10 % ethanol, about 15 % ethanol, about 20 % ethanol, about 25 % ethanol, about 30 % ethanol, about 35 % ethanol, about 40 % ethanol, about 50 % ethanol, about 70% ethanol.

Preferably, the Light Green stain utilized by the present method is about 4 % (w/v) in 20 % ethanol (when used with fixed and mounted tissue samples). The concentration of Light Green can be 10 folds less for cytological samples (i.e. 0.4% in 2% ethanol).

Fast Green can be used at a concentration of about 0.02 % [weight per volume (w/v)], about 0.1 % (w/v), about 0.2 % (w/v), about 0.3 % (w/v), about 0.4 % (w/v), about 0.5 % (w/v), about 0.6 % (w/v), about 0.7 % (w/v) about 0.8 % (w/v), about 1 % (w/v), about 2 % (w/v), about 3 % (w/v), about 4 % (w/v), about 5 % (w/v), about 6 % (w/v), about 7 % (w/v), about 8 % (w/v), about 9 % (w/v) or about 10 % (w/v).

The Fast Green solution can be a water and/or ethanol-based and can include about 0.01 % ethanol, about 5 % ethanol, about 10 % ethanol, about 15 % ethanol, about 20 % ethanol, about 25 % ethanol, about 30 % ethanol, about 35 % ethanol, about 40 % ethanol, about 50 % ethanol, about 70% ethanol.

Preferably, the Fast Green stain utilized by the present method is about 1% in 20% ethanol.

Staining the urinary system cell sample with Light/Fast Green can be performed by applying the Light/Fast Green on the urinary system cell sample, adding it thereto, or by dipping, soaking and/or incubating the urinary system cell sample in a vessel containing same.

Staining the urinary system cell sample with Light/Fast Green is effected for a time period which enables differential staining of normal cells in green, e.g. for at least 10 seconds, at least about 30 seconds, at least about 1 minute or about 30 seconds to 10 minutes.

Preferably, staining with Light/Fast Green is effected following staining with New Fuchsin and optionally Alcian Blue.

Staining with New Fuchsin and optionally Alcian Blue and with Light/Fast Green is effected as follows. The cells are first stained with New Fuchsin and then washed (e.g. with ethanol) to remove excess stain. The cells are then stained with Alcian Blue and washed. Finally, cells are stained with Light/Fast Green and washed (see Example 1 for a more detailed description). Use of New Fuchsin together with Light/Fast Green provides color contrast (red vs. green) due to the different affinity of the stains to cell components. New Fuchsin has more affinity for cancer and precancerous cells (conditioned as described herein), while Light/Fast Green has more affinity for normal cell (conditioned as described herein). The use of Alcian Blue reduces non-specific background, stains mucins and prevents rapid fading of the stain.

Thus, the present approach enables identification of abnormal cells as well as differentiation between abnormal and normal cells based on color staining differences.

When using a single stain such as New Fuchsin, a cell stained predominantly dark or intense red is identified herein as an abnormal cell, i.e. a positive result (for a pathology) whereas a cell stained predominantly light red to pink is identified herein as a normal cell, i.e. a negative result.

When using the double staining approach, a cell stained predominantly red (any shade/tone of red, e.g. pink/light red to violet or dark red is identified herein as an abnormal cell, i.e. a positive result (for a pathology) whereas a cell stained predominantly green/blue (any shade of green or blue or combinations thereof) is identified herein as a normal cell, i.e. a negative result.

Tissue samples from bladders subjected to the conditioning and double staining steps described herein were analyzed and the results are presented in Example 1 of the Examples section which follows.

Briefly, the present conditioning and staining approach results in an almost uniform pink/red cytoplasmic color of TCC cells in histological and cytological samples both low and high grade. Normal urothelium uniformly stain green. A relatively small number of areas of TCC stained green or mix (red/green) shows low Ki67 index in contrast with red areas of TCC shows high Ki67 index.

The urinary system cells stained with New Fuchsin and/or Light/Fast Green can be further stained with Hematoxylin (e.g., Hematoxylin Gill solution) for a staining period of about 10 seconds to about 9 minutes in order to counterstain the cell nuclei. This is preferably effected following fixation, prior to application of the Ficus extract. This stain highlights additional nuclear ultra structural features.

Additional stains and dyes for identifying biochemical components (e.g. immunostains such as anti-Ki67) or molecular components (e.g. polynucleotide-specific probes) can be used to further establish cell type, cell state and cell origin (especially when obtained from a urine sample). Microscopic analysis can also be used to type the cell origin. Renal tubular epithelial cells are typically larger than granulocytes, contain a large round or oval nucleus and normally slough into the urine in small numbers. Transitional epithelial cells from the renal pelvis, ureter, or bladder have more regular cell borders, larger nuclei, and smaller overall size than squamous epithelium. Renal tubular epithelial cells are smaller and rounder than transitional epithelium, and their nucleus occupies more of the total cell volume.

Thus, the present invention provides a method of identifying abnormal urinary system cells in a urine cell sample. The present invention provides a unique staining approach which is highly effective in uniquely staining preconditioned abnormal urinary system cells.

Although the basic concept of conditioning and double staining of cells has been previously described by the present inventors, use of such an approach for staining urothelial cells required calibration in both the conditioning and staining steps.

While reducing the present invention to practice, the present inventors discovered that the staining approach described in WO2007/102146 was not effective in producing the desired differential staining of urothelial cells. Calibration of this previously described double staining approach to detection of abnormal urothelial cells resulted in appreciably longer staining times with New Fuchsin and Light Green. Effective New Fuchsin staining requires incubation times which are at least twice as long as those described in WO2007/102146, while effective Light Green staining requires incubation times which are at least five times longer than the incubation times described in WO2007/102146.

The present approach can be utilized for any urinary system pathology associated with the presence of abnormal cells.

One pathology which can be identified using the present approach is bladder cancer. As is described in the Examples section which follows, the present approach proved highly effective in detecting early stage TCC in bladder tissue (Example 1). Since the chances of surviving bladder cancer dramatically increase with early detection, the present approach provides physicians with an important diagnostic tool in the fight against bladder cancer.

Thus, according to another aspect of the present invention there is provided a method of identifying in a subject (e.g. human) a bladder pathology which is characterized by presence of cell abnormalities. Preferably, such a bladder pathology is bladder cancer and the subject is suspected of having, or is predisposed to, such a pathology.

The method is effected by contacting urinary system cells (urine sample) with a Ficus plant extract thereby conditioning the abnormal cells for identification. Following fixation and conditioning the cells are stained with a basic and acidic dye (preferably with New Fuchsin and Light/fast Green as described above). Following staining, the cell sample is subjectively or objectively analyzed as described herein to identify a bladder cell or cells with a cytoplasm which is stained pink to red/violet. Such a cell is considered abnormal and indicative of a bladder pathology such as bladder cancer.

The cell sample can then be further analyzed morphologically or tested for the presence of cancer-specific markers.

The urine sample can be obtained from midstream urine or collected during cystoscopy. The sample can be fixed (for example with 50 % ethanol), and then processed using cytospin, a membrane collection method, or a liquid based cytology preparation method.

The cell sample is analyzed for both color and morphology. Color analysis is preformed as described herein, green cytoplasmic color implies negative for malignancy, and pink to red cytoplasmic color implies positive for malignancy.

TCC morphological criteria are determined as known in the art. Briefly, low-grade papillary urothelial carcinomas are characterized by an orderly appearance both architecturally and cytologically. The cells are evenly spaced and cohesive. There is minimal but definite evidence of nuclear atypia consisting of scattered hyperchromatic nuclei, infrequent mitotic figures predominantly toward the base, and mild variation in nuclear size and shape. High-grade papillary urothelial cancers contain cells that may be dyscohesive with large hyperchromatic nuclei. Some of the tumor cells show frank anaplasia. Mitotic features, including atypical ones, are frequent. Dysplasias were identified according following criteria: Change of urothelial polarization, variation in nuclear size, nuclear crowding.

The agents of the present invention described herein can be included in a diagnostic kit/article of manufacture preferably along with appropriate instructions for use and labels indicating regulatory approval for use in diagnosing and staging bladder cancer, monitoring for disease recurrence, screening of patients at risk.

Such a kit can include the above described conditioning and staining agents and instructions for use in staining specific cell preparation types (slides, suspensions, smears), as well as instructions for interpreting the results. A web site address providing further information, troubleshooting, image databases and diagnostic services (for uploaded images) can also be provided.

As used herein the term "about" refers to ± 10 %.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub combination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non limiting fashion.

### EXAMPLE 1

### Detection of Bladder Cancer using the present approach

A study was designed to test the ability of the present approach to detect abnormal cells in bladder biopsies with a known pathology. The goal of the present study was to calibrate and optimize the cell conditioning step as well as the staining steps of the present staining approach so as to enable accurate detection of abnormal bladder cells, and in particular enable early diagnosis of bladder cancer.

### Materials and Methods

### Sample collection

Bladder biopsies (archived paraffin blocks) from normal bladder, superficial bladder cancer - Ta, T1, Tis (low grade) and muscle invasive cancer (high grade) were collected and processed as described below. A total of 73 samples were received, 15 samples were not included in the study. All samples received were with a known pathology diagnosis performed at Rabin Medical Center.

Each block was processed by a pathological laboratory to prepare slides for staining. Slides from each block were stained as described below and the staining results were compared to the pathology results of the samples. For each case, one slide was stained by the H&E method for comparative purposes.

### H&E Staining

Paraffin-embedded tissue samples were sectioned at 4 microns, and mounted on standard microscopic slides. Deparaffinization was accomplished as follows: immersions in xylene 100%, twice (3 minutes each); ethanol 100%, twice (3 minutes each); immersion in ethanol 95%, twice (3 minutes each); DIW (3 minutes). Sections were stained with hematoxylin, Dye-1, (1 minute), washed in running tap water until the water was clear, and then washed for 10 seconds in running DIW. Sections were then stained in eosin, Dye-C, (1-2 minutes) and washed in tap water and DIW, as above. For cover-slipping, the sections were dehydrated in an ascending series of ethanol concentrations (50%, 70%, 80%, 95%, 100% twice), cleared in xylene 100% (3 - 4 times), and then mounted in Permount, or an equivalent commercial product.

### Staining of bladder biopsies

Staining procedures were effected in a slide container. The volume of the solutions used was adjusted to the container size. For deparaffinization, slides were heated to 70°C for 15 minutes and then immersed in xylene 100%, twice (3 minutes each); ethanol 100%, twice (3 minutes each); ethanol 95%, twice (3 minutes each); DIW (3 minutes). Sections were stained with hematoxylin (1 minute), washed in running tap water until the water was clear, and then washed for 10 seconds in running DIW. Sections were then incubated in plant extract solution (4 minutes), washed for 10 seconds in running DIW, in 0.5 % New Fuschin in ethanol 20% (2 minutes), washed as above, in 4 % Light Green in ethanol 20% (4 minutes) and washed again. For cover-slipping, sections were air-dried for 30 minutes, dipped 3 times in xylene 100% and mounted with Entellan.

### Sample Analysis

Each stained sample was analyzed as follows:
(i) Cell color - presence of green/blue colored cells (normal cells), red colored cells (potentially cancer cells) or both. A description of the colors was prepared (mostly green, small amount of red cells, etc).
(ii) Morphological analysis of the cells in the sample was also performed. As indicated above, an important advantage of the present staining approach is that it allows morphological analysis.

An alignment of color and cell morphology was performed and recorded to establish the correlation between cell morphology and cell color. Additional measures were recorded, including color intensity, color shading, spread of colored cells, etc. These measures were analyzed against the other study measures (i.e. cell morphology).

### Staining Calibration

The staining protocol was calibrated for optimal incubation parameters, using staining solutions of varying concentrations, different staining sequences and different incubation times. After establishing optimal parameters, an optimized staining approach was generated and tested in order to verify reproducibility. Following several rounds of calibration and testing, the staining approach of the present invention provided highly repetitive results. The calibrated staining protocol was then used for staining of additional samples.

### Results

A detailed analysis of resultant staining for each case was prepared and compared to both historical diagnosis as well as to the parallel stained H&E slide.

Each case (both "H&E stained slide" and the slide stained using the present approach) was first analyzed by a professional pathologist. Following completion of the analysis, the slides were transferred to an independent review and analysis by an expert pathologist.

The data from the pathology reports were used to compile the results of the present study.

Table 1 categorizes the cases into the disease groups as depicted in the clinical protocol.

**Table 1: cases categorized according to clinical diagnosis groups**

| Historical diagnosis | Number of cases |
|---|---|
| Normal | 22 |
| Low Grade TCC | 17 |
| High Grade TCC | 19 |

Table 2 compiles the data comparing results of the present staining approach to that of H&E stain, according to the original historical diagnosis:

**Table 2: Diagnostic agreement rates for the present approach and H&E on bladder biopsies**

| | Normal (22 cases) | | Low Grade TCC (17 cases) | | High Grade TCC (19 cases) | |
|---|---|---|---|---|---|---|
| Pathologist | the present approach | H&E | the present approach | H&E | the present approach | H&E |
| 1 | 19/22 ^{a, b} (86.4%) | 19/22 ^{a,} b (86.4%) | 16/17 ^{a, d} (94.1%) | 16/17 ^{a, d} (94.1%) | 18/19 ^{a, e} (94.7%) | 18/19 ^{a, e} (94.7%) |
| 2 | 20/22 ^{a, c} (90.9%) | 20/22 ^{a, c} (90.9%) | 17/17 (100%) | 17/17 (100%) | 16/20 ^{a, f} (80%) | 16/20 ^{a, f} (80%) |

| | | | | | | |
|---|---|---|---|---|---|---|
| a - Number of new diagnoses in agreement with original diagnosis per total number examined. b-Three cases (historical Normal Urinary Bladder) were diagnosed as dysplasia by a pathologist after examination of both H&E slide and the slide stained using the present approach. c - Two cases were diagnosed respectively as Low Grade and dysplasia by a pathologist after examination of both H&E slide and the slide stained using the present approach. d - One case (historical Low Grade TCC) was diagnosed as Normal by a pathologist after examination of both H&E slide and the slide stained using the present approach. e- Two cases (historical High Grade TCC) were diagnosed respectively as Hyperplasia and Low Grade TCC by a pathologist after examination of both H&E slide and the slide stained using the present approach. f -One case and three cases (all historical diagnosis High Grade TCC), were diagnosed respectively as Hyperplasia (suspicious Low Grade TCC) and Low Grade TCC by a pathologist after examination of both H&E slide and the slide stained using the present approach. | | | | | | |

Analysis of the results showed that for each pathologist, diagnosis using the slides stained with the present approach matched that of the H&E slides. Clearly, H&E staining is non specific and primarily highlights cell and tissue morphology. Thus, diagnosis of a specific disease by analysis of H&E staining requires thorough investigation of the morphology and should be performed by an expert specializing in pathology, specifically, the pathology of said disease. Advantageously, diagnosis based on the approach of the present invention is highly specific and does not require unique skills and expertise. Moreover, diagnosis based on the approach of the present invention is straightforward and rapid thereby it is time and cost effective. In addition, the staining approach of the present invention maintains tissue and cell morphology thereby allows comparing the stained slides with adjacent slide analyzed by other histological means.

In addition to the analysis of cases, an analysis of tissue regions was also performed. This is possible since in most cases there is more than one distinct region for analysis. This analysis was performed by a pathologist by carefully marking and analyzing each region and comparing the morphological diagnosis of the region to the color diagnosis.

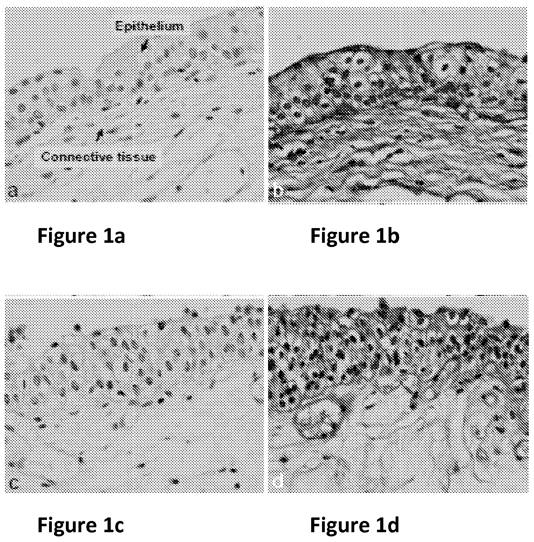

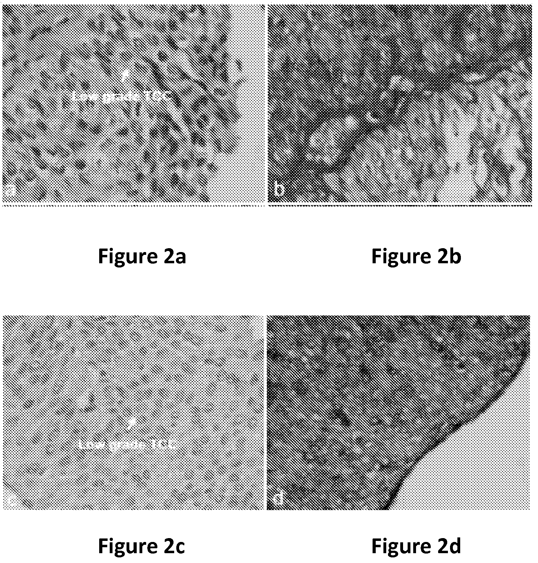

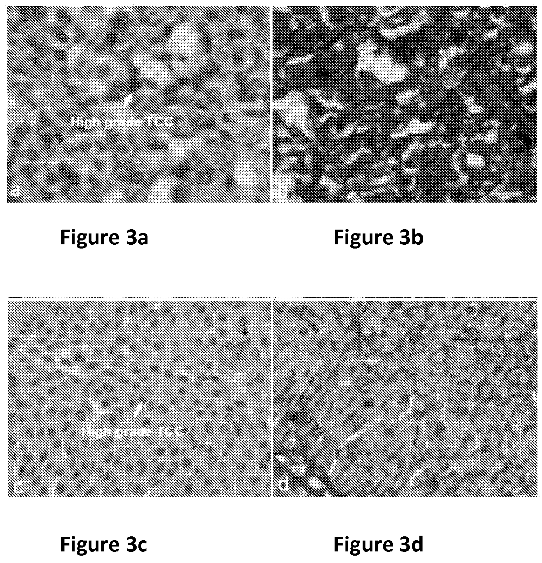

A total of 250 regions from 58 slides were analyzed. Normal regions (n=70, green cytoplasm = positive correlation) included 78.6% of regions with green cytoplasm, 14.3% of regions with red cytoplasm and 7.1% of regions with mixed color cytoplasm. Neoplastic regions (dysplastic + Low Grade TCC + High Grade TCC) (n= 180, red cytoplasm = positive correlation) included 15.6% of regions with green cytoplasm, 78.9% of regions with red cytoplasm and 5.6% of regions with mixed color cytoplasm. The results are presented in Figures 1-3, 5 and 6.

The present staining approach enabled identification of clear morphological features, thus enabling correlation between color and morphology. The above described region analysis provided a good match between the morphology of each region, and the color as displayed following the present staining approach. An important observation is that in a significant number of regions, there was a mixed color of red and green cells, or even within the cells cytoplasm. This provides another level of color difference.

Thus, the present study succeeded in calibrating and optimizing a previously described staining approach for detection of abnormal bladder cells. The calibrated staining approach of the present invention maximized the differential staining properties and enabled excellent morphological visualization capabilities. This dual analysis trait was able to confer non-inferiority to existing methods tested in this study. A high correlation between color and morphology was found.

These observations conclusively show that the present staining approach which is facilitated by the cell conditioning approach of the present invention can be used to accurately diagnose bladder cancer even in early stages.

### EXAMPLE 2

### Anti-Ki67 immunostaining

Anti-Ki67 immunostaining was used in order to better understand color morphology analysis and the appearance of a significant number of a mixed color population.

The expression of the human Ki67 protein is strictly associated with cell proliferation. During interphase, the antigen can be exclusively detected within the nucleus, whereas in mitosis most of the protein is relocated to the surface of the chromosomes. The fact that the Ki67 protein is present during all active phases of the cell cycle (G(1), S, G(2), and mitosis), but is absent from resting cells (G(0)), makes it an excellent marker for determining the so-called growth fraction of a given cell population. The fraction of Ki67-positive tumor cells (the Ki67 labeling index) is often correlated with the clinical course of the disease. (J Cell Physiol. 2000 Mar;182(3):311-22.)

### Materials and Methods

### Sample collection and Ki67 immunostaining

Paraffin sections of the bladder biopsies described in Example 1 were used for Ki67 staining. The stain was effected as known in the art, using an anti-Ki67 Rabbit monoclonal antibody (Thermo Fisher Scientific) with a Ventana machine (benchmark/XT ISH#712482). The resultant stain was compared to a duplicate slide stained using the present approach.
In addition, slides were also double stained with Ki67 immunostain and the present approach. Double staining was achieved by initial staining with anti-Ki67, as described above, and then treating the slide as described below. Calibration and optimization of the stain was performed as described in Example 1.

### A double stain of Ki67 and the present approach

Deparaffinized, Ki67-stained slides were incubated in plant extract solution (2 minutes) and washed for 10 seconds in running DIW. Slides were then dipped 5 times in 0.05 % New Fuschin in ethanol 2%, washed as above, dipped 5 times in 0.4 % Light Green in ethanol 2% and washed again. For cover-slipping, sections were air-dried for 30 minutes, dipped 3 times in xylene 100% and mounted with Entellan.

### Results

A total of 7 bladder biopsies with historical diagnosis of low grade TCC were stained for Ki67. The results were compared with those obtained using the present approach. Three parameters were considered: cell morphology, cell color and Ki67 index.
Numerous slides included multiple regions; the total number of analyzed regions was 67. The result of the staining analysis is provided in Table 3 below.

**Table 3: double staining with the present approach and anti-Ki67**

| Region diagnosis | Region color | # of regions | Ki67 index |
|---|---|---|---|
| Low grade | red | 41 | High |
| Low grade | mix | 4 | Low |
| Low grade | green | 2 | Low |
| Dysplasia | red | 4 | High |
| Metaplasia | red | 2 | High |
| Metaplasia | mix | 3 | Low |
| Normal | green | 7 | Low |
| Normal | green | 3 | High |
| Normal | red | 1 | Low |

Analysis revealed a very high correlation between the color of the present staining and the Ki67 index.

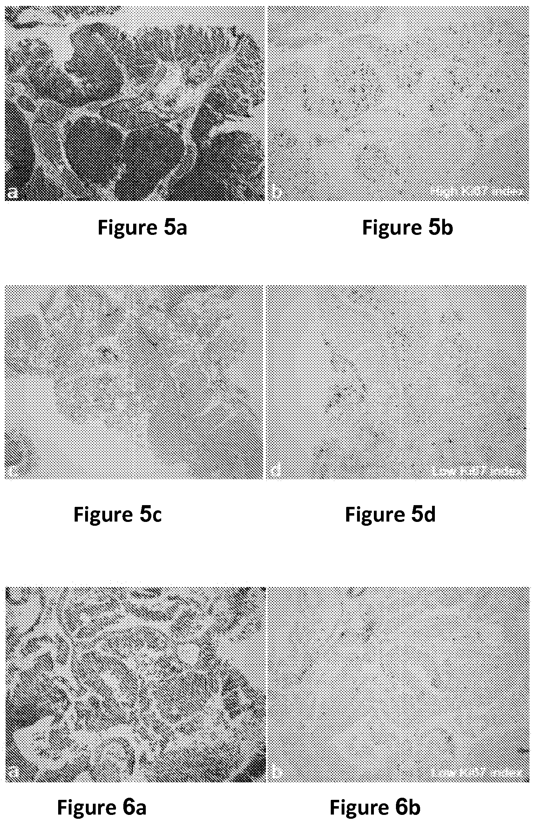

A perfect match was observed for low grade regions, all the red stained regions exhibited a high Ki67 index, whereas the green stained regions exhibited a low Ki67 index. The color mixed regions (regardless of phenotype) exhibited a low Ki67 index, while dysplastic regions, which are abnormal, stained red and exhibited a high Ki67 index. The results are presented in Figures 5-6.

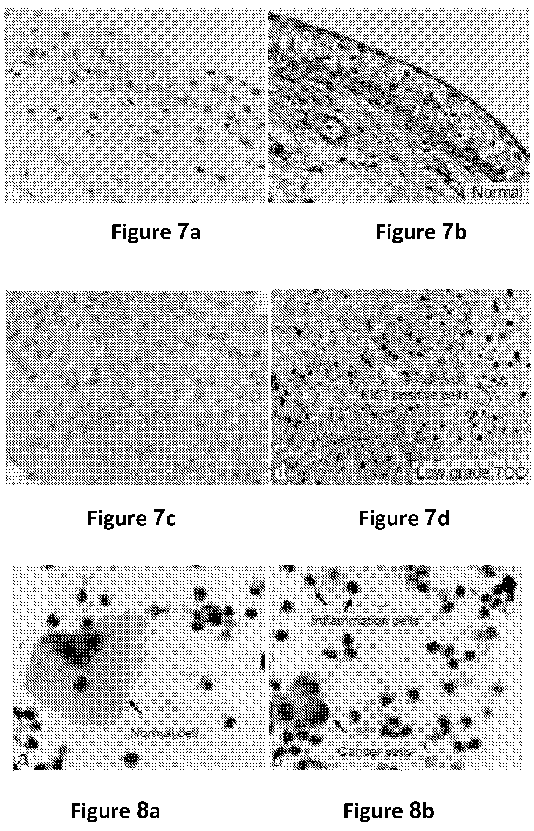

In addition to separately analyzing slides stained with Ki67 or the present approach, slides double stained with anti-Ki67 and the present approach were also analyzed. Figures 7b and 7d illustrate the results of such double-stained slides in TCC biopsies. A combination of color staining (as provided by the present approach) and a Ki67 index provides a robust and unique tool which can be especially helpful in cases that are difficult to diagnose, borderline cases. In addition, preservation of morphology, an important feature of the method of the present invention, provides an additional screening tool.

### EXAMPLE 3

### Defection of cancer cells in urine using the present approach

Urine contains epithelial cells that are shed from the urinary tract. Urine cytology evaluates the urinary sediment for the presence of cancerous cells from the urinary tract in a convenient, non-invasive manner. The sensitivity of urine cytology is low (reviewed in Mod Pathol. 2009 Jun;22 Suppl 2:S53-9), and there is a real need to develop new tests that can be used in bladder cancer screening. The goal of the present study was to calibrate and optimize the cell conditioning step as well as the staining steps of the present staining approach so as to enable accurate detection of abnormal urinary tract cells in a urine sample.

### Materials and Methods

### Sample collection

Urine samples from subjects with a known diagnosis were collected and processed by a pathological laboratory to prepare slides for staining using the cytospin method. Slides from each sample were stained as described below. Calibration and optimization of the stain was performed as described in Example 1.

### Staining of urine cells

Cytospin slides were fixed in TCA 10% (1 hour) and washed for 10 seconds in running DIW. Cells were stained with hematoxylin (1 minute), washed in running tap water until the water was clear, and then washed for 10 seconds in running DIW. Cells were then incubated in plant extract solution (4 minutes), washed for 10 seconds in running DIW, in 0.5 % New Fuschin in ethanol 20% (30 seconds), washed as above, in 0.4 % Light Green in ethanol 2% (1 minute and 40 seconds) and washed again. For cover-slipping, sections were air-dried for 30 minutes, dipped 3 times in xylene 100% and mounted with Entellan.

### Sample Analysis

Stained slides were viewed under the microscope and analyzed as described in Example 1. Briefly, normal and malignant cells were classified according to morphological features and their color was documented. An alignment of color and cell morphology was performed and recorded to establish the correlation between cell morphology and cell color.

### Results

Cytospin slides containing urinary cells were stained with the present approach and carefully examined under the microscope. Cells comprising normal urinary bladder transitional cell epithelium had greenish blue cytoplasm (Figure 8a). In contrast, morphologically recognizable neoplastic cells exhibited red/magenta tinged cytoplasm (Figure8b). Thus, it is possible to recognize neoplasia, even at low magnification, based exclusively on tinctorial status of the epithelium.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

## Claims

1. A method of identifying abnormal urinary system cells in a urine sample, the method comprising:
fixing the urine sample comprising urinary system cells in trichloroacetic acid;
contacting the urine sample with a Ficus plant extract or one or more components thereof thereby obtaining conditioned abnormal urinary system cells; and
staining the conditioned urinary system cells with a basic dye and an acidic dye, such that a cytoplasm of the abnormal cells of the urinary system cells stains with a predominantly pink to red color.

2. The method of claim 1, wherein the Ficus plant extract is an ethanol extract of leaf tissue.

3. The method of claim 1,
wherein the Ficus plant is from the subgenus Urostigma; or
wherein the Ficus plant is Ficus elastica.

4. The method of claim 1, wherein the basic dye is New Fuchsin and the acidic dye is Light Green or Fast green.

5. The method of claim 1, wherein staining conditioned urinary system cells with New Fuchsin is followed staining the cells with Light Green or Fast green.

6. The method of claim 1,
wherein the urinary system cells are urothelium cells; or
wherein the urinary system cells are bladder cells.

7. The method of claim 1,
wherein the one or more components of the extract include one or more flavonoids; or
wherein the one or more components of the extract include one or more flavonoids and the one or more flavonoids include proanthocyanidins.

8. The method of any one or more of claims 1-5, wherein the urinary system cells are urothelial cells, wherein the conditioned cells are sequentially stained with the basic dye and the acidic dye, and wherein identifying at least one urothelial cell having a cytoplasm having a basic dye color above a pre-determined threshold is indicative of abnormal cells in the sample.

9. The method of claim 1, wherein the urinary system cells are bladder cells, and wherein identifying at least one bladder cell having a red cytoplasm above a pre-determined threshold is identifying cell abnormalities indicative of the bladder pathology.

10. The method of claim 9, further comprising analyzing a morphology of the at least one bladder cell having a predominantly pink to red cytoplasm, wherein the conditioned cells are sequentially stained with the basic dye and the acidic dye, wherein the Ficus is Ficus elastica.

11. The method of claim 10,
further comprising contacting the bladder cells with an immunostain; or
further comprising contacting the bladder cells with an anti-Ki67 immunostain.

12. The method of claim 10,
wherein the bladder pathology is bladder cancer; or
wherein the bladder pathology is transitional cell carcinoma.

## Patentansprüche

1. Verfahren zur Identifizierung abnormaler Harnwegsystem-Zellen in einer Urinprobe, welches umfasst:
Fixieren der Urinprobe mit Harnwegsystem-Zellen in Trichloressigsäure;
Inkontaktbringen der Urinprobe mit einem Ficus-Pflanzenextrakt oder ein oder mehreren Komponenten davon, wodurch konditionierte abnormale Harnwegsystem-Zellen erhalten werden; und
Färben der konditionierten Harnwegsystem-Zellen mit einem basischen Farbstoff und einem sauren Farbstoff, so dass sich das Zytoplasma der abnormalen Zellen der Harnweg-system-Zellen hauptsächlich rosa oder rot färbt.

2. Verfahren nach Anspruch 1, wobei der Ficus-Pflanzenextrakt ein Ethanolextrakt des Blattgewebes ist.

3. Verfahren nach Anspruch 1,
wobei die Ficus-Pflanze von der Untergattung Urostigma ist; oder
wobei die Ficus-Pflanze Ficus elastica ist.

4. Verfahren nach Anspruch 1, wobei der basische Farbstoff New Fuchsin und der saure Farbstoff Light Green oder Fast Green ist.

5. Verfahren nach Anspruch 1, wobei dem Färben der konditionierten Harnwegsystem-Zellen mit New Fuchsin ein Färben der Zellen mit Light Green oder Fast Green folgt.

6. Verfahren nach Anspruch 1,
wobei die Harnwegsystem-Zellen Urothel-Zellen sind; oder
wobei die Harnwegsystem-Zellen Blasen-Zellen sind.

7. Verfahren nach Anspruch 1,
wobei die ein oder mehreren Komponenten des Extrakts ein oder mehrere Flavonoide umfassen; oder
wobei die ein oder mehreren Komponenten des Extrakts ein oder mehrere Flavonoide umfassen und die ein oder mehreren Flavonoide Proanthocyanidine umfassen.

8. Verfahren nach einem der Ansprühe 1-5, wobei die Hiarnwegsystem-Zellen Urothel-Zellen sind, wobei die konditionierten Zellen nacheinander mit dem basischen Farbstoff und dem sauren Farbstoff gefärbt werden, und wobei ein Identifizieren mindestens einer Urothel-Zelle mit einem Zytoplasma mit einem basischen Farbstoff oberhalb einem bestimmten Grenzwert abnormale Zellen in der Probe anzeigt.

9. Verfahren nach Anspruch 1, wobei die Harnwegsystem-Zellen Blasenzellen sind, und wobei Identifizieren mindestens einer Blasenzelle mit rotem Zytoplasma oberhalb eines bestimmten Grenzwerts Zellabnormalitäten anzeigt, die die Blasenpathology anzeigt.

10. Verfahren nach Anspruch 9, welches weiter umfasst, Analysieren einer Morphologie der mindestens einen Blasenzelle mit einem hauptsächlich rosa bis roten Zytoplasma, wobei die konditionierten Zellen nacheinander mit dem basischen Farbstoff und dem sauren Farbstoff gefärbt werden, wobei der Ficus Ficus elastica ist.

11. Verfahren nach Anspruch 10,
welches weiter umfasst, Inkontaktbringen der Blasenzellen mit einem Immunfärbemittel; oder
welches weiter umfasst, Inkontaktbringen der Blasenzellen mit einem anti-Ki67 Immunfärbemittel.

12. Verfahren nach Anspruch 10,
wobei die Blasenpathologie Blasenkrebs ist; oder
wobei die Blasenpathologie transitionelles Zellkarzinom ist.

## Revendications

1. Procédé d'identification de cellules de système urinaire anormales dans un échantillon d'urine, le procédé comprenant :
la fixation de l'échantillon d'urine comprenant des cellules de système urinaire dans de l'acide trichloracétique ;
la mise en contact de l'échantillon d'urine avec extrait de plante de Ficus ou un ou plusieurs composants de celle-ci, ce qui permet d'obtenir des cellules de système urinaire anormales conditionnées ; et
la coloration des cellules de système urinaire conditionnées, avec un colorant basique et un colorant acide, de telle sorte qu'un cytoplasme des cellules anormales des cellules de système urinaire se colore avec une couleur principalement rose à rouge.

2. Procédé selon la revendication 1, dans lequel l'extrait de plante de Ficus est un extrait par l'éthanol de tissu de feuille.

3. Procédé selon la revendication 1,
dans lequel la plante de Ficus provient du sous-genre Urostigma ; ou
dans lequel la plante de Ficus est Ficus elastica.

4. Procédé selon la revendication 1, dans lequel le colorant basique est le New Fuchsin et le colorant acide est le Light Green ou le Fast Green.

5. Procédé selon la revendication 1, dans lequel la coloration de cellules de système urinaire conditionnées avec du New Fuchsin est suivie d'une coloration des cellules avec du Light Green ou du Fast Green.

6. Procédé selon la revendication 1,
dans lequel les cellules de système urinaire sont des cellules d'urothélium ; ou dans lequel les cellules de système urinaire sont des cellules de vessie ;

7. Procédé selon la revendication 1,
dans lequel le ou les composants de l'extrait comprennent un ou plusieurs flavonoïdes ; ou
dans lequel le ou les composants de l'extrait comprennent un ou plusieurs flavonoïdes et le ou les flavonoïdes comprennent des proanthocyanidines.

8. Procédé selon l'une quelconque d'une ou plusieurs des revendications 1 à 5, dans lequel les cellules de système urinaire sont des cellules urothéliales, dans lequel les cellules conditionnées sont colorées successivement avec le colorant basique et le colorant acide, et dans lequel l'identification d'au moins une cellule urothéliale possédant un cytoplasme ayant une couleur de colorant basique au-dessus d'un seuil prédéterminé est indicative de cellules anormales dans l'échantillon.

9. Procédé selon la revendication 1, dans lequel les cellules de système urinaire sont des cellules de vessie, et dans lequel l'identification d'au moins une cellule de vessie possédant un cytoplasme rouge au-dessus d'un seuil prédéterminé identifie des anomalies cellulaires indicatives de la pathologie de la vessie.

10. Procédé selon la revendication 9, comprenant en outre l'analyse d'une morphologie de ladite au moins une cellule de vessie possédant un cytoplasme principalement rose à rouge, dans lequel les cellules conditionnées sont colorées successivement avec le colorant basique et le colorant acide, dans lequel le Ficus est le Ficus elastica.

11. Procédé selon la revendication 10,
comprenant en outre la mise en contact des cellules de vessie avec un immuno-colorant; ou
comprenant en outre la mise en contact des cellules de vessie avec un immuno-colorant anti-Ki67.

12. Procédé selon la revendication 10,
dans lequel la pathologie de la vessie est le cancer de la vessie ; ou
dans lequel la pathologie de la vessie est un carcinome transitionnel.
